# EUROPEAN PATENT SPECIFICATION

(11) **EP 1 468 660 B1**
(45) Date of publication and mention of the grant of the patent: **21.12.2011**
(21) Application number: 03011956.4
(22) Date of filing: 27.05.2003
(51) Int. Cl.: A61F 2/04

(54) **Stent delivery system, device, and method for coating**
Stentanbringungssystem und Verfahren zum Beschichten
Système de mise en place d'un stent et procédé de revêtement

(30) Priority: 15.04.2003 US 413827
(43) Date of publication of application: 20.10.2004
(73) Proprietor: Medtronic Vascular, Inc., Santa Rosa, CA 95403 (US)
(72) Inventor: Sundar, Rangarajan, Santa Rosa, CA 95404 (US)
(74) Representative: Hughes, Andrea Michelle

(56) References cited:
- EP-A- 0 887 368
- WO-A-01/52772
- WO-A-03/026718
- US-A- 6 120 847

## Description

### TECHNICAL FIELD OF THE INVENTION

The present invention relates generally to the field of implantable medical devices. More particularly, the invention relates to a stent delivery system and device having a coating, and a method for coating the same.

### BACKGROUND OF THE INVENTION

Balloon angioplasty has been used for the treatment of narrowed and occluded blood vessels. A frequent complication associated with the procedure is restenosis, or vessel re-narrowing. Within 3-6 months of angioplasty, restenosis occurs in almost 50 percent of patients. In order to reduce the incidence of re-narrowing, several strategies have been developed. Implantable devices, such as stents, have been used to reduce the rate of angioplasty related restenosis by about half. The use of such devices has greatly improved the prognosis of these patients. Nevertheless, restenosis remains a formidable problem associated with the treatment of narrowed blood vessels.

Restenosis associated with interventional procedures such as balloon angioplasty may occur by two mechanisms: thrombosis and intimal hyperplasia. During angioplasty, a balloon is inflated within an affected vessel thereby compressing the blockage and imparting a significant force, and subsequent trauma, upon the vessel wall. The natural antithrombogenic lining of the vessel lumen may become damaged thereby exposing thrombogenic cellular components, such as matrix proteins. The cellular components, along with the generally antithrombogenic nature of any implanted materials (e.g., a stent), may lead to the formation of a thrombus, or blood clot. The risk of thrombosis is generally greatest immediately after the angioplasty.

The second mechanism of restenosis is intimal hyperplasia, or excessive tissue re-growth. The trauma imparted upon the vessel wall from the angioplasty is generally believed to be an important factor contributing to hyperplasia. This exuberant cellular growth may lead to vessel "scarring" and significant restenosis. The risk of hyperplasia associated restenosis is usually greatest 3 to 6 months after the procedure.

Prosthetic devices, such as stents or grafts, may be implanted during interventional procedures such as balloon angioplasty to reduce the incidence of vessel restenosis. To improve device effectiveness, stents may be coated with one or more therapeutic agents providing a mode of localized drug delivery. The therapeutic agents are typically intended to limit or prevent the aforementioned mechanisms of restenosis. For example, antithrombogenic agents such as heparin or clotting cascade IIb/IIIa inhibitors (e.g., abciximab and eptifibatide) may be coated on the stent thereby diminishing thrombus formation. Such agents may effectively limit clot formation at or near the implanted device. Some antithrombogenic agents, however, may not be effective against intimal hyperplasia. Therefore, the stent may also be coated with antiproliferative agents or other compounds to reduce excessive endothelial re-growth. Therapeutic agents provided as coatings on implantable medical devices may effectively limit restenosis and reduce the need for repeated treatments.

Several considerations should be made when devising a strategy for coating implantable prosthetic devices, such as stents or grafts. One consideration in coating strategy relates to surface uniformity. Ideally, coatings should be evenly applied with limited surface imperfections. Some coating strategies, however, may produce pooling of the coating material and/or dry spots. Failure to control surface uniformity may lead to inaccurate, non-uniform drug dose delivery and therapeutic variability from device to device. Therefore, it would be desirable to provide a uniform stent coating.

Another consideration in coating strategy relates to topography. It may be desirable for the coating to be disposed on certain areas of the stent. For example, stents typically have a wire mesh with open spaces formed between. Some coating strategies may leave 'bridged' material within the open spaces. Such coating bridges may break off causing complications or may prevent the device from expanding or functioning properly. As another example, only certain portions of the stent may require coating, or several coating layers may be required. As yet another example, a rough irregular coating will increase variability in drug elution and stent tracking, and increase thrombogenicity and the chance of coating damage during use. As such, it would be desirable to control the stent coating topography.

Another consideration in coating strategy relates to efficiency. It may be desirable to effectively coat the implantable device in relatively short time, with a minimal amount of coating material. Some coating strategies require lengthy steps, thereby reducing the amount of devices that can be coating within a certain period. In addition, some strategies do not utilize coating material in a complete manner thereby increasing cost. For example, coating material that is vaporized may get dispersed on areas other than the stent surface. Therefore, it would be desirable to efficiently coat the stent.

Accordingly, it would be desirable to provide a strategy for coating a stent that would overcome the aforementioned and other disadvantages.

### SUMMARY OF THE INVENTION

One aspect of the present invention provides a stent delivery system. The system includes a catheter, a balloon operably attached to the catheter, and a stent disposed on the balloon. The stent includes at least one coating, the coating having an average surface roughness of less than 75 nanometers. The coating may include a therapeutic agent and may be substantially on an outer surface of the stent. The coating may be about 1 to 150 microns thick. The coating may have an average surface roughness of about 1 to 30 nanometers.

Another aspect of the invention provides a stent device. The stent device includes a body, and at least one coating, the coating having an average surface roughness of less than 75 nanometers. The coating may include a therapeutic agent and may be substantially on an outer surface of the body. The coating may be about 1 to 150 microns thick. The coating may have an average surface roughness of about 1 to 30 nanometers.

Another aspect of the invention provides a method for coating a stent. The method includes immersing a portion of the stent into a coating liquid, and withdrawing the immersed portion of the stent from the coating liquid. The method further includes simultaneously rotating the stent with respect to the coating liquid while the stent is being immersed and withdrawn. The method further includes drying the coating liquid adhering to the stent to form a coating, the coating having an average surface roughness of less than 75 nanometers. The rotation may force the coating liquid to an outer portion of the stent. Multiple layered coatings may be applied. Immersing the stent may include controlling a stent wetting characteristic. The stent may be immersed at a rate of about 0.1 to 60.0 millimeters per second, and for a time period of about 5 seconds to 10 minutes. The stent may be rotated at a rate of about 100 to 3,500 rotations per minute during immersion. Withdrawing the stent may include controlling a stent coating thickness, wherein the coating may be about 1 to 150 microns thick. The stent may be withdrawn at a rate of about 0.1 to 60.0 millimeters per second, and rotated at a rate of about 100 to 25,000 rotations per minute. The method may further include withdrawing the immersed portion of the stent from the coating liquid at a first withdrawal rate and withdrawing the immersed portion of the stent from the coating liquid at a second withdrawal rate. The method may further include programming a control sequence, and controlling at least one of the immersion, withdrawal, and rotation based on the control sequence. The coating may have an average surface roughness of about 1 to 30 nanometers.

Another aspect of the invention provides a stent device. The stent devices includes means for immersing a portion of the stent into a coating liquid, and means for withdrawing the immersed portion of the stent from the coating liquid. The stent device further includes means for simultaneously rotating the stent with respect to the coating liquid while the stent is being immersed and withdrawn. The stent device further includes means for drying the coating liquid adhering to the stent to form a coating, the coating having an average surface roughness of less than 75 nanometers. The stent device may further include a control sequence, and means for controlling at least one of the immersion, withdrawal, and rotation based on the control sequence.

The foregoing and other features and advantages of the invention will become further apparent from the following detailed description of the presently preferred embodiments, read in conjunction with the accompanying drawings. The detailed description and drawings are merely illustrative of the invention, rather than limiting the scope of the invention being defined by the appended claims.

### BRIEF DESCRIPTION OF THE DRAWINGS

**FIG. 1** is a perspective view of a stent delivery system made in accordance with the present invention;
**FIG. 2** is a perspective view of a prior art stent compatible with the disclosed coating process of the present invention;
**FIG. 3** is a magnified view of two W-shaped elements of the stent in **FIG. 2****;** and
**FIG. 4** is a diagram of a stent coating process made in accordance with the present invention.
**FIGS. 5A-5D** show stent surfaces for stents prepared by spraying and by the stent coating process of the present invention.

### DETAILED DESCRIPTION OF THE PRESENTLY PREFERRED EMBODIMENTS

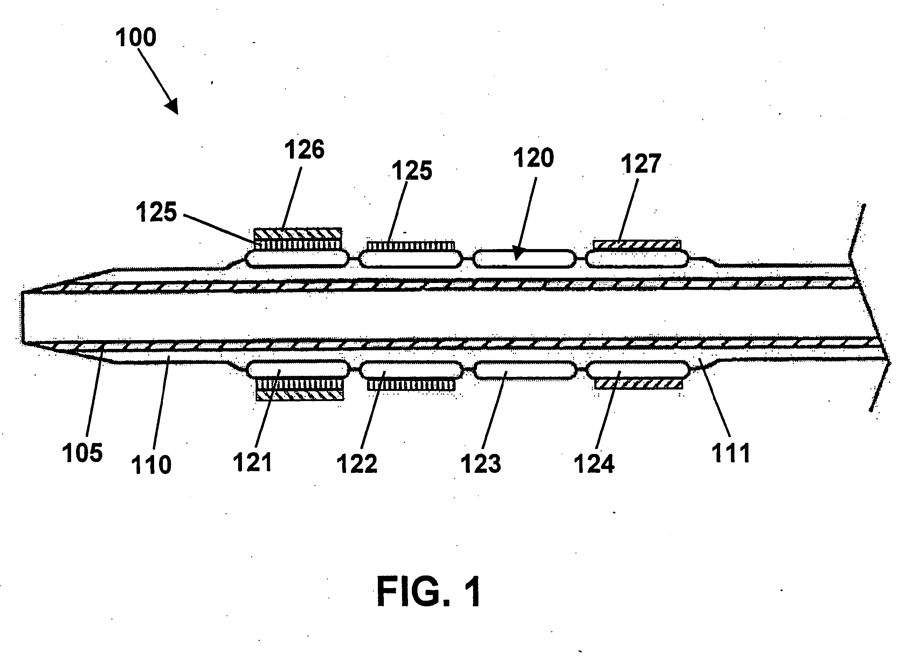

Referring to the drawings, wherein like reference numerals refer to like elements, **FIG. 1** is a perspective view of a stent delivery system made in accordance with the present invention and shown generally by numeral **100.** The stent delivery system **100** includes a catheter **105,** a balloon **110** operably attached to the catheter **105,** and a stent **120** disposed on the balloon **110.** The balloon **110,** shown in a collapsed state, may be any variety of balloons capable of expanding the stent **120.** The balloon **110** may be manufactured from any suitable material such as polyethylene, polyethylene terephthalate (PET), nylon, or the like. In one embodiment, the balloon **110** may include retention means **111**, such as mechanical or adhesive structures, for retaining the stent **120** until it is deployed. The catheter **105** may be any variety of balloon catheters, such as a PTCA balloon catheter, capable of supporting a balloon during angioplasty.

The stent **120** may be any variety of implantable prosthetic devices capable of carrying a coating known in the art. In one embodiment, the stent **120** may have a plurality of identical cylindrical stent segments placed end to end.

Four stent segments **121,122,123,** and **124** are shown, and it will be recognized by those skilled in the art that an alternate number of stent segments may be used. The stent **120** includes at least one coating applied dipping a portion of the stent **120** into a coating liquid while simultaneously rotating the stent **120.** In one embodiment, three coating layers **125, 126,** and **127** may be applied by a dip-spin coating process on various segments **121, 122,** and **124**. Segment **121** is shown having two coating layers **125**, and **126**. Segment **122** and segment **124** are shown each having one coating layer **125,** and **127,** respectively. Segment **123** is shown having no coating. The coating layers **125, 126,** and **127** are merely exemplary, and it should be recognized that other coating configurations are possible.

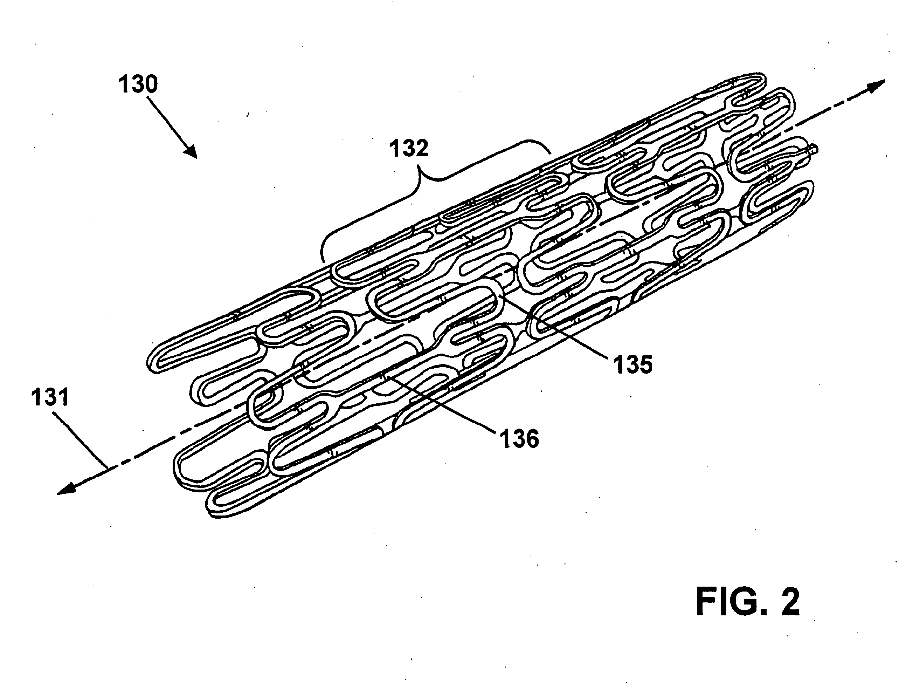

To describe the dip-spin coating process of the present invention, the following figures and description are provided. **FIG. 2** is a perspective view of a prior art stent **130** compatible with the coating process of the present invention. Those skilled in the art will recognize that numerous stents, grafts, and implantable prosthetic devices are compatible with the disclosed coating method and that the described stent **130** example is merely one illustration of the process. The stent **130** is an example of a wire-tubular hybrid stent disclosed by U.S. Patent No. 5,935,162 issued to Dang.

The stent **130** includes a generally tubular body defining a passageway extending along a longitudinal axis **131**. The stent **130** is formed from a plurality of cylindrical segments **132** arranged successively along the longitudinal axis **131**. Each of cylindrical segments **132** has a length along the longitudinal axis **131** and includes a plurality of W-shaped elements **135**. The W-shaped elements **135** open in alternating directions along the longitudinal axis **131** about the perimeter or circumference of the cylindrical segments **132**. The W-shaped elements **135** are connected to each other by a tie member **136** that is attached to center sections of each of the W-shaped elements **135**.

The stent **130** is shown in an expanded state in which the cylindrical segments **132** have been expanded radially outward from the longitudinal axis **131**. The stent **130** may be compressed into a smaller diameter for delivery within a vessel lumen at which point the stent **130** may be expanded to provide support to the vessel. The stent **130** may be of the self-expanding variety and manufactured from nickel titanium alloys and other alloys that exhibit superlastic behavior (i.e., capable of significant distortion without plastic deformation). Alternatively, the stent **130** may be designed to be expanded by a balloon or some other device, and may be manufactured from an inert, biocompatible material with high corrosion resistance. The biocompatible material should ideally be plastically deformed at low-moderate stress levels. Suitable materials include, but are not limited to, tantalum, MP35N, stainless steel, titanium ASTM F63-83 Grade 1, niobium or high carat gold K 19-22.

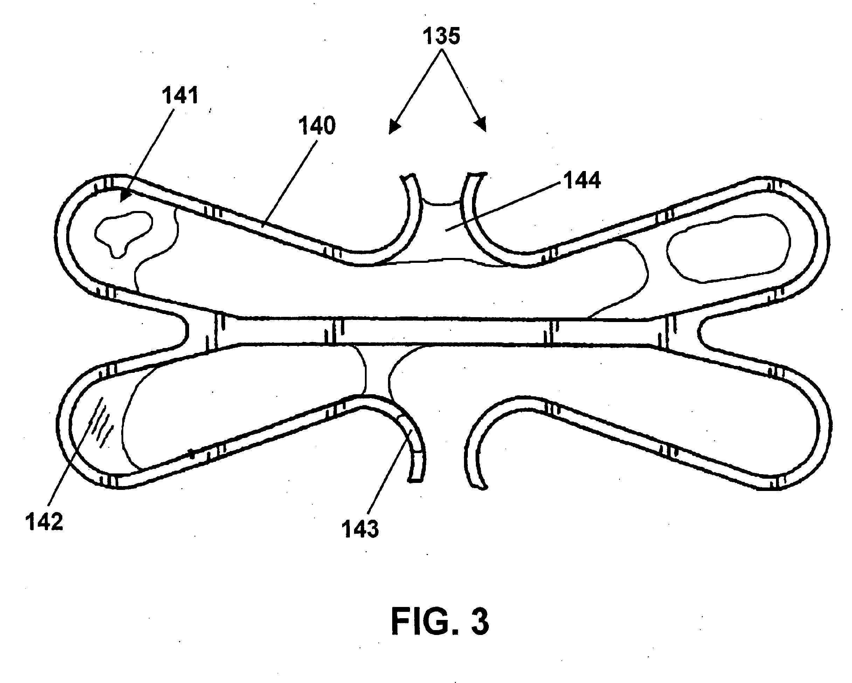

**FIG. 3** is a magnified view of two W-shaped elements **135** of the stent **130** in **FIG. 1**. The surface of the W-shaped elements is shown with a polymeric coating **140** having coating anomalies **141**. The anomalies **141** may take on numerous configurations, but common examples include pooling **142**, dry spots **143**, and bridging **144**. The anomalies **141** are typically produced during an imperfect coating process. Ideally, a coating process should produce an even coating **140** free of pooling **142**, dry spots **143**, and bridging **144**. Such coating anomalies **141** may lead to inaccurate, non-uniform drug dose delivery and therapeutic variability. In addition, anomalies such as bridging **144** may break off causing complications or may prevent the stent **130** from expanding or functioning properly. For example, bridging **144** materials may enter the bloodstream thereby releasing therapeutic agents in undesired locations. Accordingly, it is desirable to minimize coating anomalies **141**.

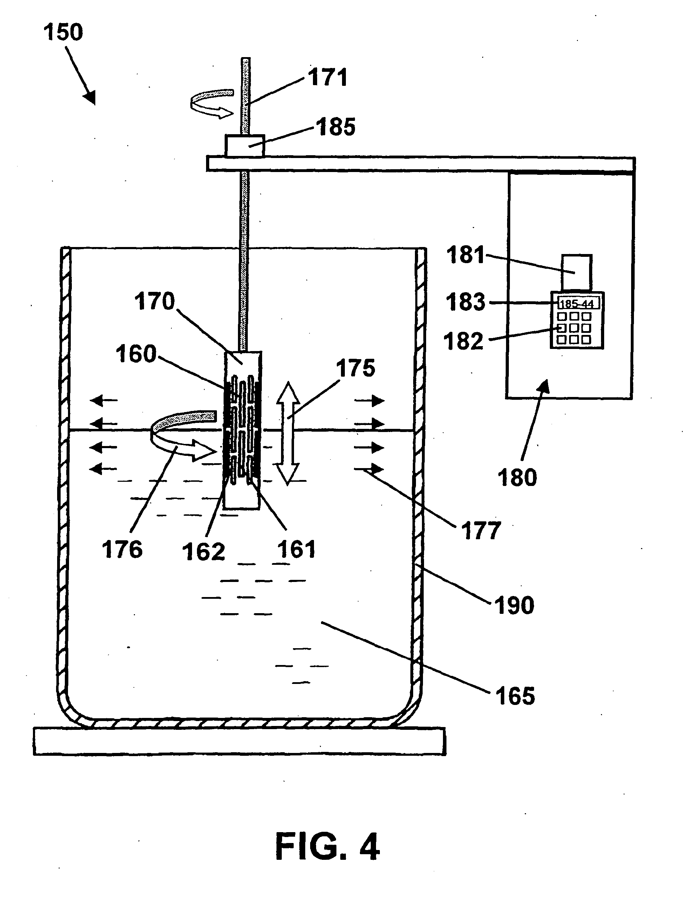

Turning now to **FIG. 4****,** a stent coating process made in accordance with the present invention is shown generally as numeral **150.** The coating process **150** provides one or more coatings on a portion of a stent device **160.** A stent device **160,** referred herein as "stent", may be any number of implantable prosthetic devices capable of carrying a coating. The stent **160** may include a body **161** and at least one coating **162** rotationally applied to a portion of the body **161**, while the body **161** is at least partially immersed in a coating liquid **165**. The stent **160** may be manufactured from a skeletal framework or mesh of material forming a tube-like structure and may be capable of self-expanding or being expanded by another device such as a balloon. The stent **160** material may include any number of metallic and polymeric biocompatible materials recognized in the art for such devices.

To apply the coating **162**, the stent **160** may be slidably mounted on a cylindrically shaped mandrel **170** that is fixably attached to a rod **171**. The stent **160**, mandrel **170**, and rod **171**, may be moved during the coating process **150** by an actuator **180**. The actuator **180** may provide vertical **175** (e.g., immersion and withdrawal) and rotational **176** movements. The actuator **180** may include a logic chip **181** programmed with a control sequence to control timing, speed, directionality, and variations of the vertical **175** and rotational **176** movement characteristics. The actuator **180** may further include a keypad **182** and display **183** for modifying and viewing portions of the control sequence, respectively. The control sequence and corresponding movement characteristics may be actuated by one or more motors **185** controlled by the logic chip **181**. The motors **185** may be of any variety of electric motors, hydraulic units, and the like, recognized in the art for providing rotational and liner movements.

The stent **160** may be dipped in the coating liquid **165** carried within a compatible container **190** (i.e., inert with respect to the coating liquid **165**). In one embodiment, the coating liquid **165** may be a polymeric solution. The polymeric solution may include one or more polymers, solvents, and therapeutic agents. The polymer may be dissolved in an appropriate solvent to provide a matrix for incorporating the therapeutic agent within the coating. Suitable polymers include, but are not limited to, urethane, polycaprolactone (PCL), polymethylmethacrylate (PMMA), polybutylmethacrylate (PBMA), and the like. Suitable solvents include, but are not limited to, acetone, ethyl acetate, tetrahydrofuran (THF), chloroform, N-methylpyrrolidone (NMP), and the like. Suitable therapeutic agents include, but are not limited to, antiangiogenesis agents, antiendothelin agents, antimitogenic factors, antioxidants, antiplatelet agents, antiproliferative agents, antisense oligonucleotides, antithrombogenic agents, calcium channel blockers, clot dissolving enzymes, growth factors, growth factor inhibitors, nitrates, nitric oxide releasing agents, vasodilators, virus-mediated gene transfer agents, agents having a desirable therapeutic application, and the like. Specific example of therapeutic agents include abciximab, angiopeptin, colchicine, eptifibatide, heparin, hirudin, lovastatin, methotrexate, streptokinase, taxol, ticlopidine, tissue plasminogen activator, trapidil, urokinase, and growth factors VEGF, TGF-beta, IGF, PDGF, and FGF. In a further embodiment, the drug coating composition may be fashioned using the drug 42-Epi-(tetrazolyl)-rapamycin, set forth in U.S. Patent No. 6,329,386 assigned to Abbott Laboratories, Abbott Park, IL. and dispersed within a coating fashioned from phosphorylcholine coating of Biocompatibles International plc, set forth in U.S. Patent No. 5,648,442.

The stent coating process **150** may begin by preparing the coating liquid **165**, such as a polymeric solution. In one embodiment, preparation of the polymeric solution may include dissolving polymer in solvent. The choice of solvent controls the ability to place the polymer into solution and, thus, should be considered. The use of solvent blends may also be considered. A blend of solvents may be preferred to ensure uniform coating **162** with a smooth surface. Solvent volatility may also be considered. Too rapid solvent evaporation may result in surface imperfections; too slow solvent evaporation may result in handling defects as well as high solvent retention. As such, the polymer-solvent solution should ideally provide a uniform coating **162**, smooth surface without major imperfections, and little to no solvent retention. In one embodiment, the polymer-solvent solution may have a solids range from about 1 to 10 percent and a viscosity of about 5 to 30 centipoise.

One or more therapeutic agents may be added to the polymeric solution. Ideally, the polymer-solvent solution should be capable of dispersing the agent evenly throughout the solution. Furthermore, the polymer-solvent solution should not chemically alter the therapeutic character of the agent. Examples of suitable polymer/solvent/therapeutic agent combinations include: polylactic acid/trichloroethane/colchicines; polyurethane/THF/taxol; (poly(D,L-lactide)/PCL)/dimethylformamide/hirudin; (poly(D,L-lactide)/polygycolide)/ethylacetate/ticlopidine; and polyethylene oxide/ethanol/heparin. These combinations are merely exemplary, and it should be recognized that other combinations are possible.

After the polymer and therapeutic agent have been dissolved in the solvent, the polymeric solution may be aged and cleared. In one embodiment, the solution may be mixed for 12 or more hours while being stirred to ensure complete dissolution and homogeneity. The solution may also be filtered with an appropriate filter to remove any impurities and haziness. Those skilled in the art will recognize that the preparation of the coating liquid **165** may vary greatly and may be contingent upon the materials used. After the coating liquid **165** has been prepared, it may be transferred to the container **190**.

The stent **160** mounted on the mandrel **170** may begin rotation as effected by the actuator motor **185**. The stent **160** may be rotated initially at a rate of about 100 to 3,500 rotations per minute (rpm). More preferably, the stent **160** may be rotated at about 500 to 700 rpm. The stent **160** may then be immersed in the coating liquid **165** at a rate of about 0.1 to 60.0 millimeters per second (mm/sec). More preferably, the stent **160** may be immersed at a rate of about 0.5 to 15.0 millimeters per second. The immersion may continue until the stent body **161** portion requiring coating has been immersed. The stent **160** may be immersed for a total time of about 5 seconds to 10 minutes. More preferably, the stent **160** may be immersed for about 5 to 20 seconds. The immersion time may control a stent wetting characteristic; the characteristic relating to how completely the stent is covered with the coating liquid **165.** Ideally, the stent **160** should be evenly "wetted" free from any dry spots. A lower viscosity coating liquid **165** generally require shorter immersion time. The temperature of the coating liquid **165** affects its viscosity and, therefore, may also be a factor in determining immersion time.

Once the stent **160** has been immersed, the stent **160** may begin withdrawal wherein it is rotated at about 100 to 25,000 rpm. More preferably, the stent **160** may be rotated during withdrawal at a rate of about 500 to 3,000 rpm. The rate of rotation during withdrawal may influence the uniformity of the coating **162.** For example, too slow rotation during withdrawal may lead to coating pooling; too fast rotation may excessively remove coating material. The stent **160** may be withdrawn at a rate of about 0.1 to 25.0 mm/sec. More preferably, the stent **160** may be withdrawn initially at 0.1 mm/sec and then at 0.7mm/sec providing a uniform coating **162.** The withdrawal rate may control a coating thickness. Generally, faster withdrawal rates produce greater coating thickness. The coating **162** may be in the range of about 1 to 150 microns thick. More preferably, the coating **162** may be about 5 to 30 microns thick. The rotating stent **160** may experience a significant centrifugal force **177**. The coating liquid **165** may be forced substantially from an inner to an outer surface portion of the stent **160**. As a result, the bulk of the coating **162** and integral therapeutic agent may be positioned proximate to where the stent **160** contacts the vessel wall. This topography may provide efficient coating liquid **165** utilization and an effective drug delivery method. Another factor affecting the coating process **150** pertains to the manner in which the coated stent **160** is dried. Ideally, the coated stent **160** should be dried to allow for a desirable rate of solvent evaporation. For example, too rapid drying causes moisture to be entrained in the coating **162** causing surface blemishes; too slow drying increases solvent retention. The drying conditions may vary greatly and are generally dependant upon the nature of the coating liquid **165** and, to a great extent, the solvent used. After the coating **162** has dried, the stent coating process **150** may be repeated to provide additional coating(s) positioned on top of, or on other locations of the stent **160**.

The described stent coating process **150** may provide a coating **162** that is controllable, uniform, efficient, and free from bridging and pooling anomalies. The process **150** may be optimized by customizing the timing, speed, directionality, and variations of the vertical **61** and rotational **62** movement characteristics. These characteristics may be adjusted based on the nature of the stent **160** and coating liquid **165** used. The concurrent spinning and dipping of the stent **160** may speed the rate at which the coating **162** is applied. In addition, the process **150** may provide efficient utilization of coating-liquid **165.** Accordingly, the process **150** may reduce the time and cost of conventional coating strategies.

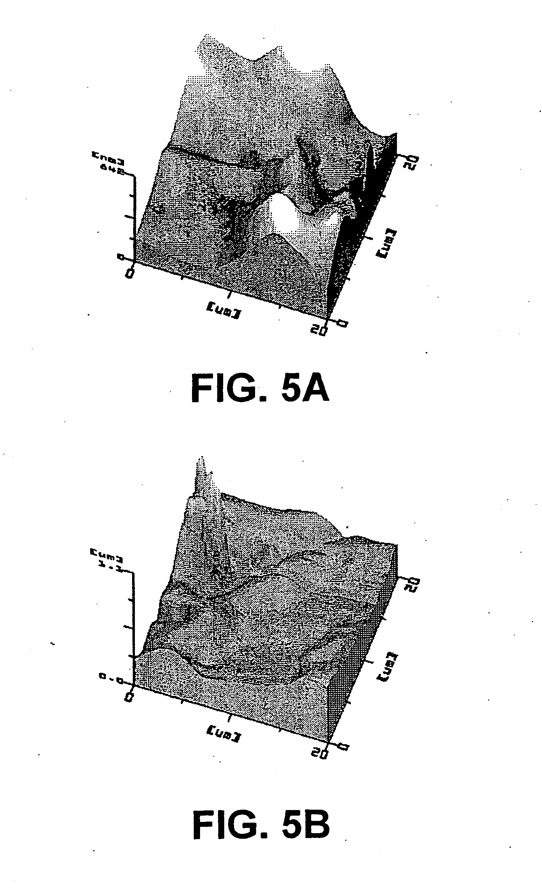

**FIGS. 5A-5D** show stent surfaces for stents prepared by spraying and by the stent coating process of the present invention. The figures illustrate the improvement in surface smoothness possible with the method of the present invention. A smooth stent coating is less thrombogenic than a rough stent coating. During manufacture, a smooth stent coating provides better control and precision in determining the amount of drug or therapeutic agent in the stent coating, regulating the amount of drug absorbed for coatings that are drug loaded by absorption, and regulating coating thickness for multiple layered coatings. During implantation, a smooth stent coating provides increased contact area, retaining the stent on the balloon more firmly and tracking (seating) the stent at the implant location more precisely. During use, a smooth stent coating provides more control of drug elution. In addition, a smooth stent coating avoids coating damage because the smooth stent coating lacks the peaks and rough spots that may be broken off when the stent is implanted through the customary tortuous path.

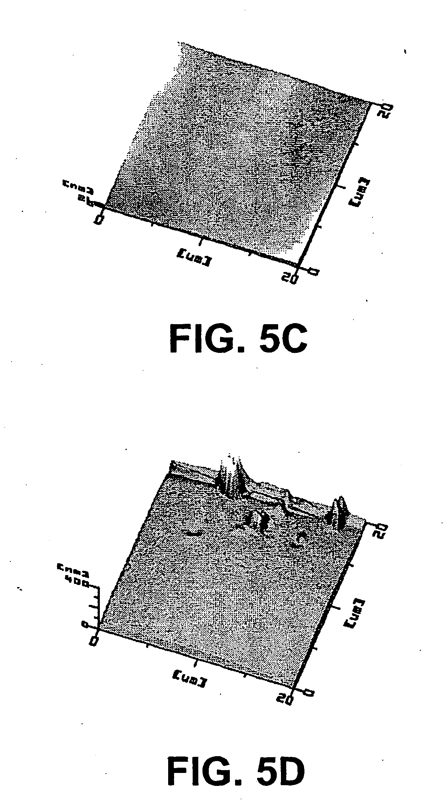

The data for the stent surfaces were collected using an atomic force microscope (AFM) with nanometer resolution. The stent coatings of **FIGS. 5A & 5B** are baseline coatings for comparison prepared by the conventional method of spraying the coating on the stent. The stent coatings of FIGS. **5C & 5D** were prepared by dipping and rotating the stent according to the present invention. The stent coatings for **FIGS. 5A-5D** were applied to a production stent made of 316L stainless steel covered with a 0.1 to 0.2 micron thick epoxy primer. The stent coatings for **FIGS. 5A-5D** were formed from a coating liquid comprising in part polybutylmethacrylate (PBMA) dissolved in chloroform.

The coating liquid forming the stent coatings of **FIGS. 5A & 5B** was sprayed on the epoxy primed base stent. The coating liquid was applied manually using a pneumatic atomizer at a distance of 2 to 10 centimeters and a flow rate of 1 to 5 cubic centimeters per minute.

The coating liquid forming the stent coatings of **FIGS. 5C & 5D** was applied to the epoxy primed base stent with the dipping and rotating method of the present invention. The base stent was rotated at 700 revolutions per minute and immersed in the coating liquid at 10 millimeters per second (mm/sec). The stent was immersed for 10 seconds, and then withdrawn from the coating liquid at 40 mm/sec. The stent was rotated at 700 revolutions per minute for 5 seconds after the stent was fully withdrawn from the coating liquid. After drying, the coating thickness was about 1.5 microns.

The average surface roughness and maximum peak heights for the stent coatings applied by the dipping and rotating method were much less than the stent coating applied by the spray method. The spray baseline cases of **FIGS. 5A & 5B** had respective average surface roughness (Ra) of 142 nanometers and 77 nanometers, with respective peak heights of 842 nanometers and 1.15 micrometers. The dipping and rotating cases of **FIGS. 5C & 5D** had respective average surface roughness (Ra) of 2.35 nanometers and 16.3 nanometers, with respective peak heights of 26.5 nanometers and 400 nanometers.

The test results presented in **FIGS. 5A-5D** are illustrative only and are not intended to limit the scope of the present invention. Similar results would be expected for different base stent materials; coating liquids containing different polymers, co-polymers, and solvents; and for different method parameters, such as rotation rate and immersion time.

While the embodiments of the invention disclosed herein are presently considered to be preferred, various changes and modifications can be made without departing from the scope of the invention as defined by the claims. For example, the stent configuration is not limited to any particular stent design. In addition, the coating liquid composition and coating process movement characteristics may be varied considerably while providing a desirable coating.

## Claims

1. A stent comprising:
a body (161); and
at least one coating (162), **characterised by** the coating having an average surface roughness of less than 75 nanometer.

2. The stent of claim 1 wherein the coating includes a therapeutic agent.

3. The stent of claim 1 or 2, wherein the coating is substantially on an outer surface of the stent.

4. The stent of any preceding claim, wherein the coating comprises a thickness of 1 to 150 microns.

5. The stent of any preceding claim wherein the coating has an average surface roughness of about 1 to 30 nanometers.

6. A stent delivery system (100) comprising:
a catheter (105);
a balloon (110) operably attached to the catheter; and
a stent (120) as claimed in any preceding claim, disposed on the balloon.

7. A method for coating a stent comprising:
immersing a portion of the stent (160) into a coating liquid;
withdrawing the immersed portion of the stent from the coating liquid;
simultaneously rotating the stent with respect to the coating liquid while the stent is being immersed and withdrawn; and
drying the coating liquid adhering to the stent to form a coating, the coating having an average surface roughness of less than 75 nanometers.

8. The method of claim 7 wherein the rotation forces the coating liquid to an outer portion of the stent.

9. The method of claim 7 or 8 further comprising applying multiple layered coatings (125, 126, 127).

10. The method of claim 7, 8 or 9 wherein immersing the stent comprises controlling a stent wetting characteristic.

11. The method of any of claims 7 to 10 wherein the stent is immersed at a rate of 0.1 to 60.0 millimeters per second.

12. The method of any of claims 7 to 11 wherein the stent is immersed for a time period of between 5 seconds and 10 minutes.

13. The method of any of claims 7 to 12 wherein the stent is rotated during immersion at a rate of 100 to 3,500 rotations per minute.

14. The method of any of claims 7 to 13 wherein withdrawing the stent comprises controlling a stent coating thickness.

15. The method of claim 14 wherein the stent coating thickness comprises a thickness of 1 to 150 microns.

16. The method of any of claims 7 to 15 wherein the stent is withdrawn at a rate of 0.1 to 60.0 millimeters per second.

17. The method of any of claims 7 to 16 wherein withdrawing the immersed portion of the stent from the coating liquid comprises withdrawing the immersed portion of the stent from the coating liquid at a first withdrawal rate and withdrawing the immersed portion of the stent from the coating liquid at a second withdrawal rate.

18. The method of any of claims 7 to 17 wherein the stent is rotated during withdrawal at a rate of 100 to 25,000 rotations per minute.

19. The method of any of claims 7 to 18 further comprising:
programming a control sequence; and
controlling at least one of the immersion, withdrawal, and rotation based on the control sequence.

20. The method of any of claims 7 to 19 wherein the coating has an average surface roughness of about 1 to 30 nanometers.

21. A stent device comprising:
means (170, 171, 180) for immersing a portion of the stent into a coating liquid (165);
means (170, 171, 180) for withdrawing the immersed portion of the stent from the coating liquid;
means (180) for simultaneously rotating the stent with respect to the coating liquid while the stent is being immersed and withdrawn; and
means for drying the coating liquid adhering to the stent to form a coating, the coating having an average surface roughness of less than 75 nanometers.

22. The device of claim 21 further comprising:
a control sequence; and
means (182, 183) for controlling at least one of the immersion, withdrawal, and rotation based on the control sequence.

## Patentansprüche

1. Stent mit:
einem Rumpf (161); und
wenigstens einer Beschichtung (162), **dadurch gekennzeichnet, dass** die Beschichtung eine mittlere Oberflächenrauhigkeit von weniger als 75 Nanometern aufweist.

2. Stent nach Anspruch 1, bei dem die Beschichtung einen therapeutischen Wirkstoff aufweist.

3. Stent nach Anspruch 1 oder 2, bei dem sich die Beschichtung im Wesentlichen auf einer äußeren Oberfläche des Stents befindet.

4. Stent nach einem der vorstehenden Ansprüche, bei dem die Beschichtung eine Dicke von 1 bis 150 Mikron bzw. Mikrometern aufweist.

5. Stent nach einem der vorstehenden Ansprüche, bei dem die Beschichtung eine mittlere Oberflächenrauhigkeit von etwas 1 bis 30 Nanometern aufweist.

6. Stentabgabesystem (100) mit:
einem Katheter (105);
einem Ballon (110), der in Wirkverbindung an dem Katheter angebracht ist; und
einem Stent (120) nach einem der vorstehenden Ansprüche, der auf dem Ballon angeordnet ist.

7. Verfahren zur Beschichtung eines Stents, das umfasst:
Eintauchen eines Abschnitts des Stents (160) in eine Beschichtungsflüssigkeit;
Herausziehen des eingetauchten Abschnitts des Stents aus der Beschichtungsflüssigkeit;
gleichzeitiges Drehen des Stents in Bezug auf die Beschichtungsflüssigkeit, während der Stent eingetaucht und herausgezogen wird; und
Trocknen der Beschichtungsflüssigkeit, die an dem Stent anhaftet, um eine Beschichtung zu bilden, wobei die Beschichtung eine mittlere Oberflächenrauhigkeit von weniger als 75 Nanometern, aufweist.

8. Verfahren nach Anspruch 7, bei dem die Drehung die Beschichtungsflüssigkeit auf einen äußeren Abschnitt des Stents zwingt.

9. Verfahren nach Anspruch 7 oder 8, das ferner das Aufbringen mehrschichtiger Beschichtungen (125, 126, 127) umfasst.

10. Verfahren nach Anspruch 7, 8 oder 9, bei dem das Eintauchen des Stents das Steuern einer Stentbenetzungscharakteristik umfasst.

11. Verfahren nach den Ansprüchen 7 bis 10, bei dem der Stent mit einer Rate von 0,1 bis 60,0 Millimetern pro Sekunde eingetaucht wird.

12. Verfahren nach einem der Ansprüche 7 bis 11, bei dem der Stent für einen Zeitraum zwischen 5 Sekunden und 10 Minuten eingetaucht wird.

13. Verfahren nach einem der Ansprüche 7 bis 12, bei dem der Stent während des Eintauchens mit einer Rate von 100 bis 3500 Umdrehungen pro Minute gedreht wird.

14. Verfahren nach einem der Ansprüche 7 bis 13, bei dem das Herausziehen des Stents das Steuern einer Stentbeschichtungsdicke umfasst.

15. Verfahren nach Anspruch 14, bei dem die Stentbeschichtungsdicke eine Dicke von 1 bis 150 Mikron bzw. Mikrometern umfasst.

16. Verfahren nach einem der Ansprüche 7 bis 15, bei dem der Stent mit einer Rate 0,1 bis 60 Millimetern pro Sekunde herausgezogen wird.

17. Verfahren nach einem der Ansprüche 7 bis 16, bei dem das Herausziehen des eingetauchten Abschnitts des Stents aus der Beschichtungsflüssigkeit das Herausziehen des eingetauchten Abschnitts des Stents aus der Beschichtungsflüssigkeit mit einer ersten Herausziehrate und das Herausziehen des eingetauchten Abschnitts des Stents aus der Beschichtungsflüssigkeit mit einer zweiten Herausziehrate umfasst.

18. Verfahren nach einem der Ansprüche 7 bis 17, bei dem der Stent während des Rausziehens mit einer Rate von 100 bis 25.000 Umdrehungen pro Minute gedreht wird.

19. Verfahren nach einem der Ansprüche 7 bis 18, das ferner umfasst:
Programmieren einer Steuersequenz; und
Steuern des Eintauchens, des Herausziehens und/oder der Umdrehung auf Grundlage der Steuersequenz.

20. Verfahren nach einem der Ansprüche 7 bis 19, bei dem die Beschichtung eine mittlere Oberflächenrauhigkeit von etwa 1 bis 30 Nanometern aufweist.

21. Stentvorrichtung mit:
Mitteln (170, 171, 180) zum Eintauchen eines Abschnitts des Stents in eine Beschichtungsflüssigkeit (165);
Mitteln (170, 171, 180) zum Herausziehen des eingetauchten Abschnitts des Stents aus der Beschichtungsflüssigkeit;
Mitteln (180) zum gleichzeitigen Drehen des Stents in Bezug auf die Beschichtungsflüssigkeit, während der Stent eingetaucht und herausgezogen wird; und
Mitteln zum Trocknen der Beschichtungsflüssigkeit, die an dem Stent anhaftet, um eine Beschichtung zu bilden, wobei die Beschichtung eine mittlere Oberflächenrauhigkeit von weniger als 75 Nanometern aufweist.

22. Vorrichtung nach Anspruch 21, die ferner aufweist:
eine Steuersequenz; und
Mittel (182, 183) zum Steuern des Eintauchens, des Herausziehens und/oder der Drehung auf Grundlage der Steuersequenz.

## Revendications

1. Endoprothèse ou stent comportant :
un corps (161) ; et
au moins un revêtement (162), **caractérisée en ce que** le revêtement a une rugosité de surface moyenne inférieure à 75 nanomètres.

2. Endoprothèse selon la revendication 1, dans laquelle le revêtement inclut un agent thérapeutique.

3. Endoprothèse selon la revendication 1 ou 2, dans laquelle le revêtement est sensiblement sur une surface extérieure de l'endoprothèse.

4. Endoprothèse selon l'une quelconque des revendications précédentes, dans laquelle le revêtement présente une épaisseur de 1 à 150 microns.

5. Endoprothèse selon l'une quelconque des revendications précédentes, dans laquelle le revêtement a une rugosité de surface moyenne d'environ 1 à 30 nanomètres.

6. Système de délivrance par endoprothèse (100) comportant :
un cathéter (105) ;
un ballon (110) attaché au cathéter de manière opérationnelle ; et
une endoprothèse (120) telle que revendiquée dans l'une quelconque des revendications précédentes, disposée sur le ballon.

7. Procédé d'enduction d'une endoprothèse, comportant les étapes consistant à :
immerger une partie de l'endoprothèse (160) dans un liquide de revêtement ;
retirer la partie immergée de l'endoprothèse du liquide de revêtement ;
faire tourner simultanément l'endoprothèse par rapport au liquide de revêtement pendant que l'endoprothèse est immergée et retirée ; et
sécher le liquide de revêtement adhérant à l'endoprothèse pour former un revêtement, le revêtement ayant une rugosité de surface moyenne inférieure à 75 nanomètres.

8. Procédé selon la revendication 7, dans lequel la rotation force le liquide de revêtement jusqu'à une partie extérieure de l'endoprothèse.

9. Procédé selon la revendication 7 ou 8, comportant en outre l'application de multiples revêtements disposés en couches (125, 126, 127).

10. Procédé selon la revendication 7, 8 ou 9, dans lequel l'immersion de l'endoprothèse comporte la commande d'une caractéristique de mouillage d'endoprothèse.

11. Procédé selon l'une quelconque des revendications 7 à 10, dans lequel l'endoprothèse est immergée à une vitesse de 0,1 à 60,0 millimètres par seconde.

12. Procédé selon l'une quelconque des revendications 7 à 11, dans lequel l'endoprothèse est immergée pendant une période de temps comprise entre 5 secondes et 10 minutes.

13. Procédé selon l'une quelconque des revendications 7 à 12, dans lequel l'endoprothèse est mise en rotation pendant l'immersion à une vitesse de 100 à 3 500 tours par minute.

14. Procédé selon l'une quelconque des revendications 7 à 13, dans lequel le retrait de l'endoprothèse comporte la commande d'une épaisseur de revêtement d'endoprothèse.

15. Procédé selon la revendication 14, dans lequel l'épaisseur de revêtement d'endoprothèse présente une épaisseur de 1 à 150 microns.

16. Procédé selon l'une quelconque des revendications 7 à 15, dans lequel l'endoprothèse est retirée à une vitesse de 0,1 à 60,0 millimètres par seconde.

17. Procédé selon l'une quelconque des revendications 7 à 16, dans lequel le retrait de la partie immergée de l'endoprothèse du liquide de revêtement comporte le retrait de la partie immergée de l'endoprothèse du liquide de revêtement à une première vitesse de retrait et le retrait de la partie immergée de l'endoprothèse du liquide de revêtement à une seconde vitesse de retrait.

18. Procédé selon l'une quelconque des revendications 7 à 17, dans lequel l'endoprothèse est mise en rotation pendant le retrait à une vitesse de 100 à 25 000 tours par minute.

19. Procédé selon l'une quelconque des revendications 7 à 18, comportant en outre les étapes consistant à :
programmer une séquence de commande ; et
commander au moins une étape parmi l'immersion, le retrait et la rotation, sur la base de la séquence de commande.

20. Procédé selon l'une quelconque des revendications 7 à 19, dans lequel le revêtement a une rugosité de surface moyenne d'environ 1 à 30 nanomètres.

21. Dispositif d'endoprothèse comportant :
des moyens (170, 171, 180) pour immerger une partie de l'endoprothèse dans un liquide de revêtement (165) ;
des moyens (170, 171, 180) pour retirer la partie immergée de l'endoprothèse du liquide de revêtement ;
des moyens (180) pour faire tourner simultanément l'endoprothèse par rapport au liquide de revêtement pendant que l'endoprothèse est immergée et retirée ; et
des moyens pour sécher le liquide de revêtement adhérant à l'endoprothèse pour former un revêtement, le revêtement ayant une rugosité de surface moyenne inférieure à 75 nanomètres.

22. Dispositif selon la revendication 21 comportant en outre :
une séquence de commande ; et
des moyens (182, 183) pour commander au moins une étape parmi l'immersion, le retrait et la rotation, sur la base de la séquence de commande.
